# EUROPEAN PATENT APPLICATION

(11) **EP 4 714 439 A1**
(43) Date of publication of application: **25.03.2026**
(21) Application number: 24201999.0
(22) Date of filing: 23.09.2024
(51) Int. Cl.: A61K 31/352, A61K 36/185, A61K 36/605, A61P 31/00, A61P 31/16

(54) **ANTIVIRAL PLANT EXTRACT COMBINATIONS FOR TREATING RESPIRATORY TRACT INFECTIONS**

(71) Applicant: DSM IP Assets B.V., 6221 BE Maastricht (NL)
(72) Inventor: HUG, Hubert Paul, 79418 Schliengen (DE); CIVILETTO, Gabriele, 4303 Kaiseraugst (CH); KUENZLI, Katharina, 4303 Kaiseraugst (CH)
(74) Representative: dsm-firmenich IP

(57) **Abstract**

The present invention relates to an antiviral plant extract combination for use in the treatment or prevention of a respiratory tract infection. In particular, the invention provides a combined preparation, a combination, and a composition, comprising a first component comprising Quercetin and a second component comprising a plant extract from Elderberry and/or Mulberry leaf.

## Description

### FIELD OF THE INVENTION

The present invention relates to antiviral plant extract combinations for use in the treatment or prevention of a respiratory tract infection, in particular respiratory tract infection caused by viral infection.

### BACKGROUND OF THE INVENTION

Viral infections remain a major global cause of morbidity and mortality. Influenza is an infectious respiratory disease in humans caused by influenza viruses. Influenza viruses infect 5-15% of the global population annually, causing 3-5 million cases of severe illness and accounting for 290,000-650,000 deaths each year due to respiratory illness. There are four types of influenza viruses: types A, B, C, and D. Severe acute respiratory syndrome (SARS), a viral respiratory disease caused by a SARS-associated coronavirus, is also recognized as a global life-threatening respiratory infection. Notably, severe acute respiratory syndrome coronavirus type 2 (SARS-CoV-2), a member of the same family as severe acute respiratory syndrome coronavirus (SARS-CoV) and Middle East respiratory syndrome coronavirus (MERS-CoV), emerged as a highly transmissible pathogen causing the COVID-19 pandemic and accounted for several million direct or indirect deaths at the height of the pandemic. SARS-CoV-2 virus is a single-stranded positive-strand RNA virus with an envelope structure. Like influenza viruses, SARS-CoV-2 virus is now exhibiting a seasonal pattern, resulting in waves of illness worldwide. The emergence of new viruses or variants of existing viruses pose increased risks that potentially threaten human health and public safety. Hence, there is an urgent need for solutions that fight viral infections.

Various strategies have been developed to counter viral infections. Recently, there is a trend of developing combinations of antiviral agents that are effective against different viruses. However, the antiviral drugs have been reported to cause adverse effects, particularly in case of administration of combination drugs. Furthermore, antivirals often show limited efficacy, and the inappropriate use of these drugs may increase the risk of antiviral resistance. One of the efforts to obtain alternative therapies is using natural products or plants (such as plant extracts and natural small molecules) as sources, as they have been in use for medicinal purposes since ancient times and are known for their antiviral properties and more tolerable side effects.

For some plant extracts, antiviral activity is known.

Elderberry has been traditionally used in the treatment of influenza and common cold infections. In an *in vitro* study on antiviral activity of elderberry fruit *(Sambucus nigra* L.) extracts, Roschek B Jr et al. have reported that the elderberry fruit extracts can inhibit human Influenza A (H1N1) infection (see, Roschek B Jr et al. Elderberry flavonoids bind to and prevent H1N1 infection in vitro. Phytochemistry. 2009 Jul; 70(10):1255-61. PMID 19682714). Furthermore, Kinoshita E et al. have confirmed the *in vivo* effect of Elderberry (*Sambucus nigra* L.) extract on stimulating the immune response and preventing viral infections (see, Kinoshita E et al., Anti-influenza virus effects of elderberry juice and its fractions. Biosci Biotechnol Biochem. 2012; 76(9):1633-8. PMID 22972323).

Mulberry *(Morus alba),* a member of the *Moraceae* family, has been known to have antiviral effects against hepatitis B and C viruses. In a study on the antiviral activities of Mulberry juice and seed against influenza viruses, a group of authors have confirmed that the presence of phenolic compounds in Mulberry seeds identified by LC-MS attribute to the significant antiviral effect against influenza viruses, suggesting Mulberry seeds can be developed as a novel plant-derived antiviral against influenza viruses (Kim H, Chung MS. Antiviral Activities of Mulberry (Morus alba) Juice and Seed against Influenza Viruses. Evid Based Complement Alternat Med. 2018 Nov 1, 2018: 2606583. PMID 30515232).

In a study from Derosa G et al. on the potential role for Quercetin as an antiviral compound it was found that Quercetin might have the theoretical capability to interfere with SARS-CoV-2 replication. However, no *in vitro* study on antiviral activity was performed (see, Derosa G et al. A role for quercetin in coronavirus disease 2019 (COVID-19). Phytother. Res. 2021 Mar;35(3):1230-1236. PMID 33034398). A follow-up review on Quercetin highlighted a wide spectrum of antiviral activities of Quercetin and its derivates and the potential use of Quercetin in virus infection prevention and treatment (see, Di Petrillo A et al. Quercetin and its derivates as antiviral potentials: A comprehensive review. Phytother. Res. 2021 Oct 28, PMID 34709675). Quercetin was also shown to exhibit antiviral activity against influenza A virus (IAV) by targeting the virus entry, which is the initial step of the viral replication cycle (see, Wu W et al. Quercetin as an Antiviral Agent Inhibits Influenza A Virus (IAV) Entry. Viruses. 2015 Dec 25 ;8(1):6. PMID 26712783).

Further aspects are described in Jacob JR et al. J Microbiol. 2007 Oct; 45(5):431-40. PMID 17978803; Thabti I et al. Molecules. 2020 Apr 18; 25(8):1876. PMID 32325742; Sayed A. et al. Food Science and Human Wellness 7, Issue 1, March 2018, Pages 91-101; Sivarajan R et al. BMC Complement Med Ther. 2022 Jul 8; 22(1):181. PMID: 35804339; and Harnett J et al. Adv Integr Med. 2020 Dec; 7(4):240-246. PMID 32864330.

The efficacy of treatments using plant extracts is often limited. There remains a need to identify a treatment that enhances the antiviral effects of individual plant-based treatments and/or addresses the limitations of antiviral drugs such as low bioavailability and side effects associated with high doses.

### SUMMARY OF THE INVENTION

Surprisingly, the inventors found that combinations comprising Quercetin and certain plant extracts exert synergistic antiviral effects against viruses causing respiratory tract infections. In particular, combinations of Quercetin and a plant extract from Mulberry leaf and/or Elderberry exhibit a synergistic increase of *in vitro* inhibition of viral infectivity of influenza A or SAR-CoV-2 viruses in the human lung cell line Calu-3, while showing only a low cytotoxicity.

The present invention provides combinations and compositions comprising Quercetin and a plant extract from Mulberry leaf and/or Elderberry for use in the treatment or prevention of a respiratory tract infection.

The invention therefore relates to the subject matter defined in the following items [1] to [49]:
[1] A combination comprising a first component and a second component for use in the treatment or prevention of a respiratory tract infection, wherein said first component comprises, or consists of, Quercetin, and said second component comprises at least one plant extract selected from the group consisting of Elderberry extract, Mulberry leaf extract, and combinations thereof.
[2] The combination for use according to item [1], wherein said combination is a composition comprising said first component and said second component.
[3] The combination for use according to item [1], wherein said combination is a kit comprising said first component and said second component.
[4] The combination for use according to item [3], wherein said first component is physically separated from said second component; and/or wherein said first component is not in admixture with said second component.
[5] The combination for use according to any one of items [1] to [4], wherein said second component comprises Elderberry extract.
[6] The combination for use according to item [5], wherein said Elderberry extract is an extract from *Sambucus nigra* L.
[7] The combination for use according to item [5], wherein said Elderberry extract is an extract from *Sambucus nigra* L. ssp. nigra, *Sambucus nigra* L. ssp. *canadensis,* and/or *Sambucus nigra* L. ssp. *cerulea.*
[8] The combination for use according to any one of items [1] to [7], wherein said second component comprises Mulberry leaf extract.
[9] The combination for use according to item [8], wherein said Mulberry leaf extract is an extract from the leaves of a plant of the genus *Morus.*
[10] The combination for use according to item [8], wherein said Mulberry leaf extract is an extract from the leaves of a plant of the species *Morus alba* L., *Morus boninensis* Koidz., *Morus cathayana* Hems!., *Morus celtidifolia* Kunth, *Morus indica* L., *Morus koordersiana* J.-F.Leroy, *Morus liboensis* S.S.Chang, *Morus macroura* Miq., *Morus microphylla* Buckley, *Morus miyabeana* Hotta, *Morus mongolica* (Bureau) C.K.Schneid., *Morus nigra* L., *Morus notabilis* C.K.Schneid., *Morus rubra* L., *Morus serrata* Roxb., *Morus trilobata* (S.S.Chang) Z.Y.Cao, and/or *Morus wittiorum* Hand.-Mazz.
[11] The combination for use according to item [8], wherein said Mulberry leaf extract is an extract from the leaves of *Morus alba.*
[12] The combination for use according to item [8], wherein said Mulberry leaf extract is an extract from the leaves of *Morus alba* L.
[13] The combination for use according to any one of the preceding items, wherein said respiratory tract infection is caused by a virus.
[14] The combination for use according to item [13], wherein said virus is selected from the group consisting of coronaviruses, influenza viruses, parainfluenza viruses, respiratory syncytial virus (RSV), adenoviruses, and rhinoviruses.
[15] The combination for use according to any one of items [13] to [14], wherein said virus is a coronavirus.
[16] The combination for use according to item [15], wherein said coronavirus is SARS-CoV-2.
[17] The combination for use according to any one of items [13] to [16], wherein said respiratory tract infection comprises COVID-19, is associated with COVID-19, accompanied by COVID-19, or causes COVID-19.
[18] The combination for use according to any one of items [13] to [14], wherein said virus is an influenza virus.
[19] The combination for use according to any one of items [13] to [14], wherein said virus is an influenza A virus.
[20] The combination for use according to item [19], wherein said influenza A virus is H1N1, H2N2, or H3N2.
[21] The combination for use according to any one of the preceding items, wherein said respiratory tract infection comprises a pneumonia, is associated with a pneumonia, is accompanied by a pneumonia, or causes a pneumonia.
[22] The combination for use according to any one of items [1] to [21], wherein said treatment or prevention comprises administering to a subject 10 mg to 1,000 mg quercetin.
[23] The combination for use according to any one of items [1] to [21], wherein said treatment or prevention comprises administering to a subject 50 mg to 500 mg quercetin.
[24] The combination for use according to any one of the preceding items, wherein said treatment or prevention comprises administering to a subject quercetin at a daily dose of 10 mg to 1,000 mg.
[25] The combination for use according to any one of the preceding items, wherein said treatment or prevention comprises administering to a subject quercetin at a daily dose of 50 mg to 500 mg.
[26] The combination for use according to any one of the preceding items, wherein said treatment or prevention comprises administering to a subject 10 mg to 1,000 mg of said mulberry leaf extract.
[27] The combination for use according to any one of the preceding items, wherein said treatment or prevention comprises administering to a subject 50 mg to 500 mg of said mulberry leaf extract.
[28] The combination for use according to any one of the preceding items, wherein said treatment or prevention comprises administering to a subject a daily dose of 10 mg to 1,000 mg of said mulberry leaf extract.
[29] The combination for use according to any one of the preceding items, wherein said treatment or prevention comprises administering to a subject a daily dose of 50 mg to 500 mg of said mulberry leaf extract.
[30] The combination for use according to any one of the preceding items, wherein said treatment or prevention comprises administering to a subject 10 mg to 1,000 mg of said elderberry extract.
[31] The combination for use according to any one of the preceding items, wherein said treatment or prevention comprises administering to a subject 50 mg to 500 mg of said elderberry extract.
[32] The combination for use according to any one of the preceding items, wherein said treatment or prevention comprises administering to a subject a daily dose of 10 mg to 1,000 mg of said elderberry extract.
[33] The combination for use according to any one of the preceding items, wherein said treatment or prevention comprises administering to a subject a daily dose of 50 mg to 500 mg of said elderberry extract.
[34] The combination for use according to any one of the preceding items, wherein said treatment or prevention comprises administering said first component and said second component simultaneously.
[35] The combination for use according to any one of items [1] to [33], wherein said treatment or prevention comprises administering said first component and said second component sequentially.
[36] The combination for use according to item [35], wherein said first component and said second component are administered within one hour.
[37] The combination for use according to item [35], wherein said first component and said second component are administered within 10 minutes.
[38] The combination for use according to any one of the preceding items, wherein said treatment or prevention comprises administering said first component and said second component for a period of up to three months, e.g. for a period of 1 to 90 days.
[39] The combination for use according to any one of the preceding items, wherein said treatment or prevention comprises administering said first component and said second component for a period of 2 to 28 days, or for a period of 3 to 14 days.
[40] The combination for use according to any one of the preceding items, wherein said treatment or prevention comprises administering said first component and said second component for a period of 3 to 10 days, or for a period of 3 to 7 days.
[41] The combination for use according to any one of the preceding items, wherein said first component is administered in the form of a liquid composition.
[42] The combination for use according to any one of the preceding items, wherein said second component is administered in the form of a liquid composition.
[43] The combination for use according to any one of items [1] to [40], wherein said first component is administered in the form of a solid composition.
[44] The combination for use according to any one of items [1] to [40] and [43], wherein said second component is administered in the form of a solid composition.
[45] The combination for use according to any one of items [1] to [40], wherein said first component is administered in the form of a semisolid composition.
[46] The combination for use according to any one of items [1] to [40] and [43], wherein said second component is administered in the form of a semisolid composition.
[47] The combination for use according to item [45] or [46], wherein said semisolid composition is selected from the group consisting of gels, gel capsules, dispersions, emulsions, syrups, and pastes.
[48] The combination for use according to item [41] or [42], and [45] or [46], wherein said first and/or said second component are administered in the form of a capsule.
[49] The combination for use according to item [43] or [44], wherein said first and/or said second component are administered in the form of a tablet or capsule.

### BRIEF DESCRIPTION OF DRAWINGS

To understand the invention, some preferred embodiments will be described in the following, by the way of non-limiting examples only, and with reference to the accompanying drawings; in which:

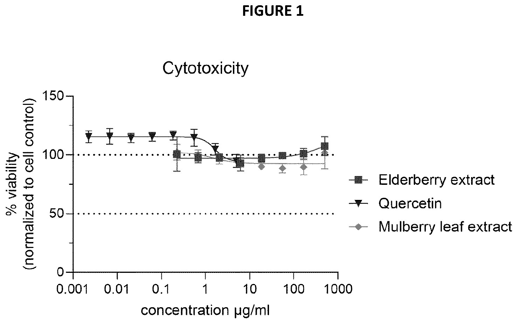

**Figure 1** shows the results of cytotoxicity studies of plant extracts on a human lung cell line (Calu-3 cells). The viability of Calu-3 cells in the presence or absence of varying concentrations of Quercetin and plant extracts from Elderberry and Mulberry leaves were measured by MTT assay.

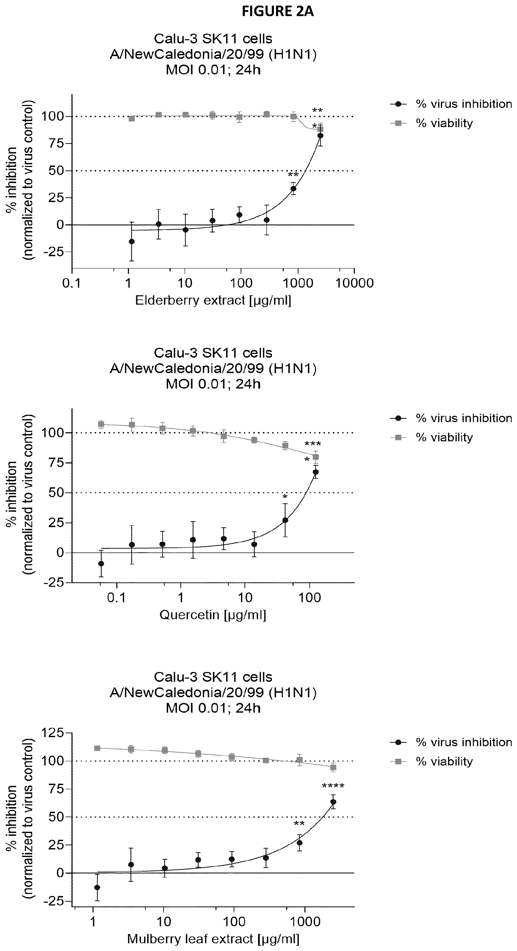

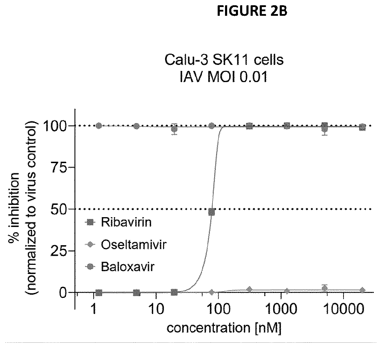

**Figure 2** shows the *in vitro* antiviral efficacy of plant extracts against influenza A virus strain H1N1. Calu-3 cells were infected with influenza A virus strain H1N1 in the presence or absence of varying concentrations of the tested plant extracts. The inhibition of viral replication was determined by immunofluorescence of N protein. (A) Quercetin (Quercefit) and plant extracts from Elderberry and Mulberry leaf; (B) Controls (Ribavirin, oseltamivir and Baloxavir).

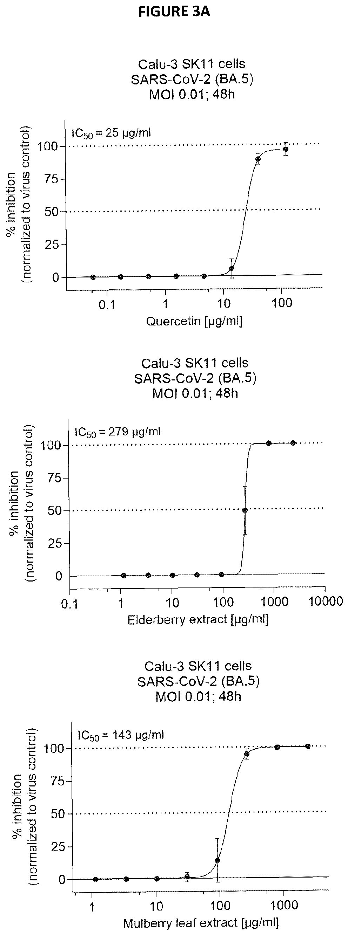

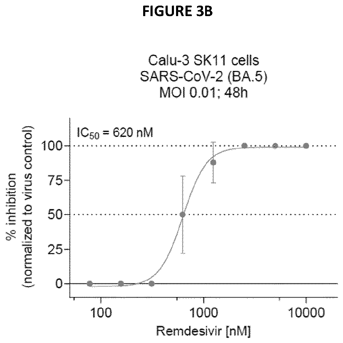

**Figure 3** shows the *in vitro* antiviral efficacy of plant extracts against SARS-CoV-2 virus (omicron BA.5 variant). Calu-3 cells were infected with SARS-CoV-2 virus (omicron BA.5 variant) in the presence or absence of varying concentrations of the tested plant extracts. The inhibition of viral replication was determined by manual cytopathic effect (SPE) readout. (A) Quercetin (Quercefit) and plant extracts from Elderberry, and Mulberry leaf; (B) Control (remdesivir).

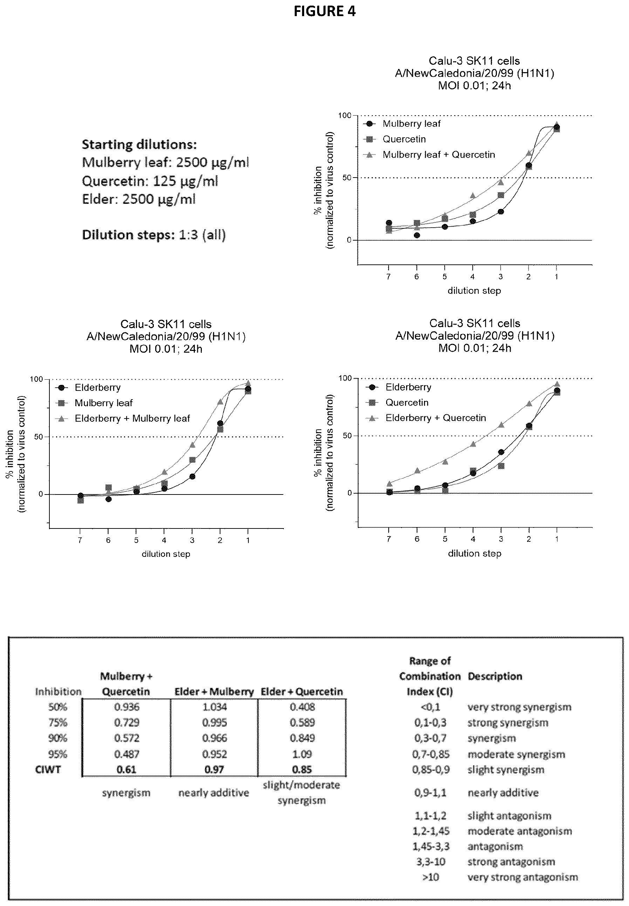

**Figure 4** shows the *in vitro* antiviral efficacy of combinations of plant extracts against the influenza A virus strain H1N1. Calu-3 cells were infected with influenza A virus strain H1N1 in the presence or absence of varying concentrations of Elderberry, Mulberry leaf, and Quercetin (Quercefit), alone or in combination. The inhibition of viral replication was determined by immunofluorescence of the N protein. Synergistic effects were evaluated using the mathematical model of Chou, 2006. Synergistic effects were evaluated using the mathematical model of Chou, 2006.

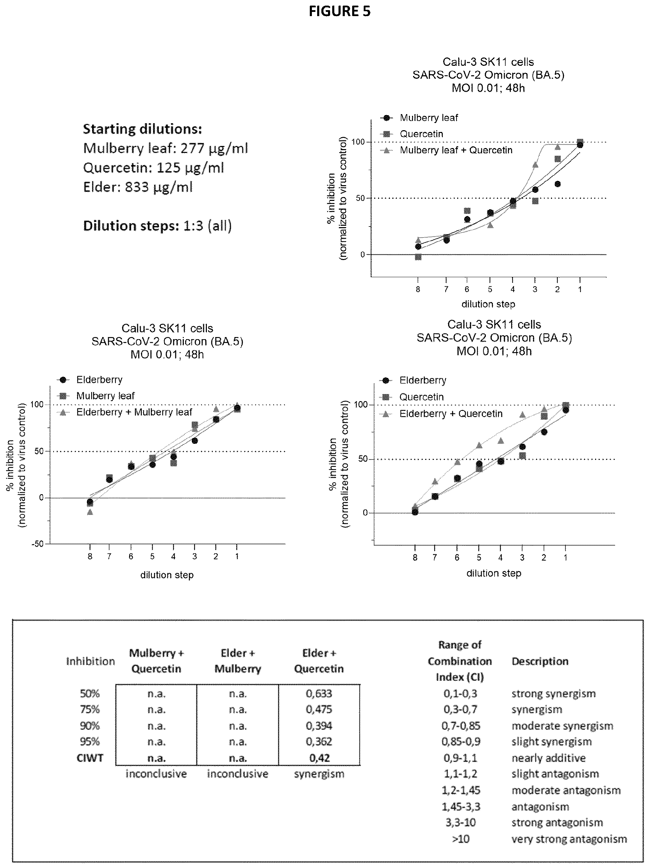

**Figure 5** shows the *in vitro* antiviral efficacy of combinations of plant extracts against SARS-CoV-2 virus (omicron BA.5 variant). Calu-3 cells were infected with SARS-CoV-2 virus (omicron BA.5 variant) in the presence or absence of varying concentrations of Elderberry, Mulberry leaf, and Quercetin (Quercefit), alone or in combination. The inhibition of viral replication was determined by immunofluorescence of the spike protein. Synergistic effects were evaluated using the mathematical model of Chou, 2006.

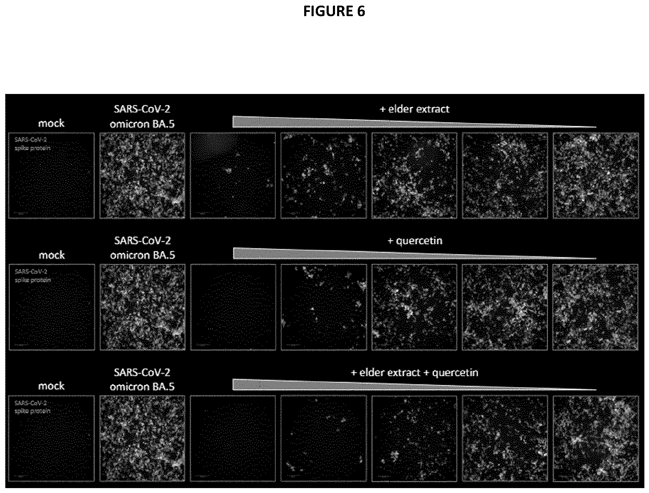

**Figure 6** shows Calu-3 cells infected with SARS-CoV-2 virus (omicron BA.5 variant) or mock-infected in the presence of the dilution steps shown in Figure 5 of Elderberry leaf extract, Quercetin (Quercefit), or the combination of Elderberry leaf extract and Quercetin (Quercefit), as indicated. The cells were stained by immunofluorescence of the spike protein.

### DETAILED DESCRIPTION OF THE INVENTION

Unless defined otherwise, all technical and scientific terms used herein have the same meaning as is commonly understood by one of skill in the art to which the subject matter herein belongs. As used in the description and the appended claims, unless specified to the contrary, the following terms have the meaning indicated below to facilitate the understanding of the present invention.

As used in the description and the appended claims, the singular forms "a", "an", and "the" include plural referents unless the context clearly dictates otherwise.

The transitional term "comprising," which is synonymous with "including" or "containing," is inclusive or open-ended and does not exclude additional, unrecited elements or method steps. By contrast, the transitional phrase "consisting of" excludes any element, step, or ingredient not specified in the claim.

The term "extract" as used herein includes compositions obtained by solvent extraction (which are also known as "plant extract") or other methods known to the skilled person. Suitable extraction solvents include alcohols such as ethanol.

The term "combination" as used herein, includes, but is not limited to, (i) combined preparations wherein the components are in one dosage unit form, e.g. compositions wherein the components are in admixture, and (ii) non-fixed combinations, where the components are physically separated. An example of a non-fixed combination is a kit of parts. The term "combination" includes the combined use of the components, wherein the components are administered separately or sequentially.

The term "liquid formulation" as used herein, refers particularly to a solution, a suspension with solid particles dispersed in a liquid, or a combination thereof, or an emulsion with liquid droplets dispersed in a liquid, or to a syrup or a syrup-like liquid. The "liquid" can be hydrophilic or lipophilic, preferably lipophilic.

The terms "semisolid formulation" and "semisolid composition", as used herein, include, but are not limited to, gels, dispersions, emulsions, syrups, syrup-like pastes, gel capsules, and pastes.

As used herein, the term "synergistic" refers to an effect of a combination which is greater than the additive effects of the components of the combination. Synergistic, additive or antagonistic effects between agents may be quantified by analysis of the dose-response curves using the Combination Index (CI) method. A CI value greater than 1,1 indicates antagonism; a CI value of 0,9-1,1 indicates an additive effect; and a CI value less than 0,9 indicates a synergistic effect. Methods for analyzing a mathematical synergy model are known in the art, see, for example, Chou TC. Theoretical basis, experimental design, and computerized simulation of synergism and antagonism in drug combination studies. Pharmacol Rev. 2006 Sep;58(3):621-81. doi: 10.1124/pr.58.3.10. Erratum in: Pharmacol Rev. 2007 Mar;59(1):124. PMID: 16968952.

The plant extracts to be used in accordance with the present invention may be obtained by any extraction method of medicinal plants known in the art. In one embodiment, the solvent for extraction is an alcohol, e.g. methanol. In another embodiment, the solvent for extraction is a mixture of ethanol with water. According to further embodiments, the solvent for extraction is a mixture of ethanol with water in an ethanol:water volume ratio ranging from 40:60 to 96:4, from 50:50 to 90:10, from 60:40 to 85:15, or from 70:30 to 80:20. According to yet another embodiment, the solvent for extraction is 100% water.

The obtained extract may be concentrated by removing the solvent, for example by subjecting the extract to reduced pressure and optionally heat. The plant extract used in accordance with this invention may be a solid product, such as a powder, or a liquid or semisolid product.

Quercetin, a natural flavonoid compound having the chemical name 2-(3,4-Dihydroxyphenyl)-3,5,7-trihydroxy-4H-1-benzopyran-4-one, is a flavonol, and is the major polyphenolic flavonoid found in various vegetables and fruits, such as berries, lovage, capers, cilantro, dill, apples, and onions. The chemical structure of quercetin in its aglycon form is shown in Formula (I)

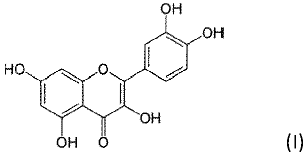

The term "quercetin" as used herein includes quercetin glycosides. Examples of quercetin glycosides include, but are not limited to, Quercetin-3-O-rutinoside, Quercetin-3-O-rhamnoside, Isoquercetin, and Quercetin-3-glucoside. In addition, the term quercetin includes quercetin derivatives having a better bioavailability and/or solubility than quercetin in its aglycon form. Such derivatives may be selected from the group consisting of quercetin hybrids, C-substituted quercetins, O-glycoside quercetins, imine quercetins, selenone quercetins, and thienone quercetins (see, Seyedeh Roya Alizadeh, Mohammad Ali Ebrahimzadeh, Quercetin derivatives: Drug design, development, and biological activities, a review, European Journal of Medicinal Chemistry, Volume 229, 2022, 114068, ISSN 0223-5234, https://doi.org/10.1016/j.ejmech.2021.114068; incorporated herein by reference). The term "quercetin" further includes prodrugs of the aglycon quercetin, such as quercetin-3-O-acyl esters (Montenegro, Lucia & Carbone, Claudia & Maniscalco, Claudio & Lambusta, Daniela & Nicolosi, Giovanni & Ventura, Cinzia & Puglisi, Giovanni. (2007). In vitro evaluation of quercetin-3-O-acyl ester as topical prodrugs. International journal of pharmaceutics. 336. 257-62. 10.1016/j.ijpharm.2006.12.003; incorporated herein by reference). The term "quercetin" also includes mixtures of quercetin and phospholipids (phytosomes) (see Kexia Zhang, Meiyu Zhang, Ziying Liu, Yuanyuan Zhang, Liqiang Gu, Gaosheng Hu, Xiaohui Chen, Jingming Jia, Development of quercetin-phospholipid complex to improve the bioavailability and protection effects against carbon tetrachloride-induced hepatotoxicity in SD rats, Fitoterapia, Volume 113, 2016, Pages 102-109, ISSN 0367-326X, https://doi.org/10.1016/j.fitote.2016.07.008; or Lu M, Qiu Q, Luo X, Liu X, Sun J, Wang C, Lin X, Deng Y, Song Y. Phyto-phospholipid complexes (phytosomes): A novel strategy to improve the bioavailability of active constituents. Asian J Pharm Sci. 2019 May;14(3):265-274. doi: 10.1016/j.ajps.2018.05.011. Epub 2018 Jul 27. PMID: 32104457; PMCID: PMC7032241; incorporated herein by reference); and/or other formulations of quercetin, e.g. nanoparticles (Xinlong Zang et al. (2021). Quercetin Nanoformulations: A Promising Strategy for Tumor Therapy. Food & Function. 12. 10.1039/D1F000851J.; incorporated herein by reference). Quercetin is commercially available from many sources and can be obtained, for example, from Indena S.p.A., Milan, Viale Ortles, n. 12 (www.indena.com).

According to one embodiment, the second component comprises, or consists of, an extract from Elderberry, preferably from one or more of the following: *Sambucus nigra* L. ssp. nigra, *Sambucus nigra* L. ssp. canadensis, *Sambucus nigra* L. ssp. *cerulea.* Elderberry is the dark purple berry of the European or black elder tree, which grows in the warmer parts of Europe, North America, Asia, and Northern Africa. An example of the Elderberry extract which can be used in the invention is commercially available from many sources and can be obtained, for example, from Anklam Extrakt GmbH (www.anklam-extrakt.de).

The Elderberry extract used in accordance with this invention preferably has a total phenols content, based on the dried extract and determined by UV-VIS spectroscopy, of at least 10% (w/w), more preferably of at least 20% (w/w), most preferably of at least 25% (w/w). The phenols content may range from about 5% to about 50%, or from about 10% to about 40%, or from about 20% to about 30% (all concentrations w/w).

The Elderberry extract used in accordance with this invention preferably has a content of total anthocyanins, as determined by HPLC and based on the dry matter, of at least 5%, more preferably of at least 10%, most preferably of at least 15% (all concentrations w/w). The content of total anthocyanins in the Elderberry extract, as determined by HPLC and based on the dry matter, may range from about 5% to about 30%, or from about 10% to about 25%, or from about 15% to about 20% (all concentrations w/w).

According to another embodiment, the second component comprises, or consists of, an extract from Mulberry leaf, preferably from the leaves of one or more of the following: *Morus alba, Morus nigra, Morus notabilis, Morus serrata, Morus celtidifolia, Morus insignis, Morus rubra,* and *Morus mesozygia.* Suitable species include *Morus alba* L., *Morus boninensis* Koidz., *Morus cathayana* Hemsl., *Morus celtidifolia* Kunth, *Morus indica* L., *Morus koordersiana* J.-F.Leroy, *Morus liboensis* S.S.Chang, *Morus macroura* Miq., *Morus microphylla* Buckley, *Morus miyabeana* Hotta, *Morus mongolica* (Bureau) C.K.Schneid., *Morus nigra* L., *Morus notabilis* C.K.Schneid., *Morus rubra* L., *Morus serrata* Roxb., *Morus trilobata* (S.S.Chang) Z.Y.Cao, or *Morus wittiorum* Hand.-Mazz. Mulberry leaf extracts which can be used in the invention are commercially available from many sources and can be obtained, for example, from Huisong Pharmaceuticals (www.huisongpharm.com).

The Mulberry leaf extracts which may be used in accordance with the present invention preferably have a total phenols content, based on the dried extract and determined by UV-VIS spectroscopy, of at least 1% (w/w), more preferably of at least 2% (w/w), most preferably of at least 3% (w/w). The phenols content may range from about 1% to about 10%, or from about 2% to about 5%, or from about 3% to about 3% (all concentrations w/w).

According to one embodiment, the invention provides a cytotoxicity counter screen to avoid the selection of cytotoxic substances, using the MTT (3-(4,5 Dimethyl thiazol-2-Yl)-2,5-Diphenyltetrazolium Bromide) assay according to the standard protocol of the supplier. The MTT assay is a colorimetric assay which is well-known in the art for determination of the cell viability and cytotoxicity. The principle of the MTT test is based on the ability of MTT to pass through the cell membrane as well as the mitochondria inner membrane of viable cells. Mitochondrial enzyme succinate dehydrogenase can reduce the tetrazolium dye MTT to its insoluble formazan product, which has purple color. The degree of formazan accumulation reflects the metabolic activity of viable cells and can be quantified by spectrophotometric means. The number of viable cells was measured by MTT as well described in the art (Luganini et al., 2008, 2010).

Provided herein are methods by which the reduction of viral infection of a host cell can be measured. The use of such methods is known in the field of virology and can be readily adopted by the skilled person to determine if an extract reduces viral infection and therefore can be used in the combination of the invention.

An immunofluorescence assay for detection of infectivity of a viable virus in a sample following cell culture infection is well-known in the art. In an embodiment, an immunofluorescent staining is performed using an antibody against the N protein. In another embodiment, an immunofluorescence staining assay is performed using an antibody against the spike protein. The viral replication is calculated by immunofluorescence results of staining of anti-N protein or anti-spike protein. In a preferred embodiment, the inhibition of viral replication may be observed by fluorescence microscope.

In a preferred embodiment, detection of infectivity of a viable virus in a sample following cell culture infection of a virus, e.g., SARS-CoV-2, is performed by using a virus-induced cytopathic effects (CPE) readout. The method can be readily performed by the skilled person using the methods disclosed herein and known in the art.

IC₅₀ measurement is a well-known measure of the potency of a substance in inhibiting a specific biological function, in this case viral infection of a host cell. It is a quantitative measure which indicates how much a particular inhibitory substance, in this case is Quercetin and a plant extract, is needed to inhibit *in vitro* a given biological process by 50%. The calculation of IC₅₀ measurements for Quercetin and plant extracts can be readily performed by the skilled person using the methods disclosed herein and known in the art.

The inventors found that Quercetin and certain plant extracts do not exert any toxicity to host cell *in vitro* and inhibit the replication of Influenza virus H1N1 strain and Corona virus SARS-CoV-2 strain after 24 hours of infection.

In one embodiment, the combination is for use in the treatment or prevention of a respiratory tract infection caused by influenza A virus infection. According to certain embodiments of the invention, the influenza A virus is H1N1, H2N2, and/or H3N2.

In another embodiment of the invention, the combination is for use in the treatment or prevention of a respiratory tract infection caused by a coronavirus, preferably by SARS-CoV-2. In a specific embodiment, the respiratory tract infection is caused by the Omicron variant of the SARS-CoV-2 virus.

In a preferred embodiment, the invention relates to a combination comprising quercetin and a plant extract from Elderberry for use in the treatment or prevention of an infection caused by influenza A virus, preferably influenza A virus H1N1.

In another preferred embodiment, the invention relates to a combination comprising quercetin and a plant extract from Mulberry leaf for use in the treatment or prevention of an infection caused by influenza A virus, preferably influenza A virus H1N1.

In yet another preferred embodiment, the invention relates to a combination comprising quercetin and a plant extract from Elderberry for use in the treatment or prevention of an infection caused by SARS-CoV-2, preferably by the omicron variant of SARS-CoV-2.

According to another aspect, the invention relates to a kit for use in the treatment or prevention of a respiratory tract infection, wherein the kit comprises the said first component and said second component.

The first component and the second component of the combination according to the present invention may be administered simultaneously, sequentially or separately over the course of treatment of the respiratory tract infection.

In one embodiment, the plant extract is a powder, a solution or a suspension or a combination thereof.

The route of administration is not particularly limited. In a preferred embodiment, the first and second components are administered orally. In another embodiment, the first and second components are administered parenterally, e.g. by intravenous injection. In yet another embodiment, the first and second components are administered by inhalation or intranasally. In the latter embodiment, the first and second components may be provided as aerosols. Methods to prepare compositions and optionally devices suitable for the respective route of administration are known to one of skill in the art.

These and other aspects of the present invention will be further appreciated upon consideration of the following Examples, which are intended to illustrate certain embodiments of the invention but are not intended to limit its scope, as defined by the claims.

### EXAMPLES

### Materials and methods

### Plant extracts

Quercetin and plant extracts were obtained from commercial sources.

Quercetin (Indena, Phospholipid SF/MD, Quercefit, Product No. 9047510, from Sophora japonica L. (Fabaceae), Lot No. 21S0154001).

Elderberry extract (Elderberry Trockenextrakt (eldosamb), Anklam, Product No. 00-115-1016-03, AEnat^{®} 1016-03, water/ethanol extract, Lot no. 20000656/0).

Mulberry leaf extract 4:1 (*Morus Alba*) (Huisong Pharmaceuticals, "internal", water extract, Lot No. MLE-MA 114-210701).

### Viruses and cell lines

SARS-CoV-2 virus (omicron BA.5 variant) was isolated from patients at the Institute for Medical Virology of the University Clinic Frankfurt am Main. H1N1 Influenza strain A virus was obtained from the Institute of Virology, Marburg, Germany. Human Calu-3 cells, derived from lung adenocarcinoma, were obtained from ATCC (HTB-55).

### Extract preparation

Dry extracts were dissolved in DMSO and diluted in culture medium at the desired concentration for the virus infection assay.

### Virus infection assay

Infection assays were carried out according to manufacturer's instructions. Calu-3 cells (lung adenocarcinoma cell line) were used as a model for influenza A virus and SARS-CoV-2 virus infection. Prior to the antiviral assays, the cells were seeded in 96 well plate. On the day of assay, maintenance medium was replaced with medium containing test compounds diluted at desired concentrations (8 dilutions per compound). Preincubation with the compounds was for approximately 1 h. Subsequently, the cells were infected with virus at a Multiplicity of infection (MOI) of 0.1. In parallel, cell plates treated only with medium containing compounds were prepared for toxicity assay. After 24 h, both assays were stopped. Viral replication was determined by immunofluorescence of N protein and cytotoxicity was evaluated by MTT assay. Three independent experiments in triplicate for each condition were performed. For each compound IC50, CC50 and SI parameters were calculated. In the case of SARS-CoV-2, viral replication was determined by immunofluorescence of the spike protein. Three independent experiments in triplicate for each condition were performed. Controls consisted of culture medium alone, culture medium with extract, and untreated noninfected cells. Furthermore, all tests were compared with plates consisting of untreated infected cells.

### Immunofluorescence assay

Immunofluorescence assays for detection of infectivity of a viable virus in a sample following cell culture infection are well-known in the art. Immunofluorescent staining was performed using an antibody against the N protein or an antibody against the spike protein. The binding of primary antibodies was detected with secondary antibodies (Sigma). The viral replication was calculated by immunofluorescence results of staining of anti-N protein or anti-spike protein, respectively, or virus-induced cytopathic effects (CPE) readout. Samples were examined using an Olympus IX70 inverted laser scanning confocal microscope, and images were captured using FluoView 300 software (Olympus Biosystems).

### Analysis of combination effects

To evaluate the combined effect between Quercetin and other plant extracts, the combination index or CI value was calculated using Chou and Talalay's algorithm (Chou et al., Adv Enzyme Regul, 22, 27-55 (1984)) as an indicative marker for the antagonistic or synergistic effect for the combination of two.

| **Range of combination index (CI)** | | | **Description** |
|---|---|---|---|
| | <0.1 | | Very strong synergism |
| | 0.1-0.3 | | Strong synergism |
| | 0.3-0.7 | | Synergism |
| | 0.7-0.85 | | Moderate synergism |
| | 0.85-0.9 | | Slight synergism |
| | 0.9-1.1 | | Nearly additive |
| | 1.1-1.2 | | Slight antagonism |
| | 1.2-1.45 | | Moderate antagonism |
| | 1.45-3.3 | | Antagonism |
| | 3,3-10 | | Strong antagonism |
| | >10 | | Very strong antagonism |

### Example 1: Cytotoxicity assay

Cytotoxicity of Quercetin (Quercefit) and all extracts were determined by the MTT assay according to the standard protocol of the supplier. Calu-3 cells in logarithmic growth phase were seeded in wells at 5,000 - 10,000 cells per well (96 total wells) and grown for 24-96 hours. After 24 hours of original seeding, Quercetin (Quercefit) and other extracts diluted at varying dilutions were added to the wells. For the cytotoxicity assay, Quercetin (Quercefit), and extracts from Elderberry and Mulberry leaf were tested. After treatment time of 48 hours, the media was removed and replaced with 100 µl of 37°C media in each well. Then, 25 µl of MTT stock solution (12 mM) was added to each well and the plate was incubated at 37°C for 4 hours. 100 µl of medium from each well was then removed and 50 µl of DMSO was added to each well and mixed thoroughly by pipetting. The mixture was incubated at 37 °C for 10 minutes and absorbance read at 570 nm. For each compound IC₅₀, CC₅₀ and SI was calculated. Three independent experiments in triplicate for each condition were performed.

The results shown in Figure 1 demonstrate that Quercetin (Quercefit) and extracts from Elderberry and Mulberry leaf were not toxic to uninfected Calu-3 cells at the concentration ranging from 0.001 to about 1,000 µg/ml.

### Example 2: Antiviral effects of plant extracts on influenza A strain H1N1 infected cells

A study on the antiviral activity of Quercetin and plant extracts from Elderberry and Mulberry leaf was carried out. Ribavirin, Baloxavir and Oseltamivir were used as controls. Oseltamivir is a specific anti-Influenza drug. Baloxavir is a highly potent anti-influenza drug, targeting the virus' cap-dependent endonuclease that is an essential enzyme in the IAV life cycle. Ribavirin is a synthetic guanosine analog with a broad-spectrum of antiviral activity. Calu-3 cells were infected with influenza A strain H1N1 using the method as indicated in the Materials and Methods. Quercetin (Quercefit) and other extracts were tested at varying dilutions. Viral replication was determined by immunofluorescence of N protein as indicated in the Materials and Methods.

The results shown in Figure 2 demonstrate that Quercetin and plant extracts from Elderberry and Mulberry leaf exhibited strong inhibition of viral infectivity at high concentrations without being toxic. Oseltamivir and Baloxavir exhibited antiviral activity on influenza A strain H1N1 infected cells while Ribavirin did not.

### Example 3: Antiviral effects of plant extracts on SARS-CoV-2 virus infected cells

Infections of Calu-3 cells with SARS-CoV-2 virus (omicron BA.5 variant) were performed as indicated in the Materials and Methods. Quercetin and plant extracts from Elderberry and Mulberry leaf were tested at varying dilutions. Viral replication was determined by manual cytopathic effect (CPE) as indicated in the Materials and Methods.

The results shown in Figure 3 demonstrate that Quercetin and plant extracts from Elderberry and Mulberry leaf exhibited strong inhibition of viral infectivity at high concentrations without being toxic.

### Example 4: Antiviral effects of combinations of extracts on influenza A virus infected Calu-3 cells

Based on the results obtained from the cytotoxicity and antiviral effects of extracts, Quercetin (Quercefit) and the extracts of Elderberry and Mulberry leaf were selected for further study on combinations of two that potentially exhibit synergistic effects on influenza A virus infected Calu-3 cells. The following combinations were tested alone and in combination of two: Mulberry leaf and Elderberry; Mulberry leaf and Quercetin (Quercefit); and Elderberry and Quercetin (Quercefit).

Quercetin (Quercefit) 125 µg/ml, and extracts from Elderberry 2,500 µg/ml and Mulberry leaf 2,500 µg/ml stock solutions were prepared. The extracts were then subjected to 3-fold serial dilutions in the culture medium (8 dilution steps).

The Calu-3 cells were infected with influenza A strain H1N1 using the method as indicated in the Materials and Methods. Quercetin (Quercefit) and extracts from Elderberry and Mulberry leaf were tested at varying dilutions. Viral replication was determined by immunofluorescence of N protein as indicated in the Materials and Methods. The effects of the combined use were evaluated by calculating a Combination Index (CI) following the method of Chou, 2006.

In Figure 4, the dose response curves of the individual extracts alone and the respective combinations of two, respectively, are plotted in dilution steps as to overlay the curves, even with different starting concentrations. In all combinations, a left shift was observed, indicating an at least additive effect. These results were analyzed using a mathematical synergy model as indicated above. The combination indices (CIs) for the respective effective concentrations (EC50, EC75, EC90, EC95) were obtained. Weighting of these different CIs (CI50 being weighted once, CI75 twice, CI90 thrice, and CI95 four times), resulted in the weighted CI (CIWT) that gave an indication of the combinations across the whole dose-range. The results revealed the synergistic effect of Quercetin (Quercefit) with the tested plant extracts on inhibition of influenza A strain H1N1 infectivity. The combination of Quercetin (Quercefit) with the extract from Mulberry leaf showed the CI value of 0.61, which is an indicative of synergistic effect. The synergistic effect of the combination of Quercetin (Quercefit) and the extract from Elderberry was also demonstrated, with an CI value of 0.85, an indicative of a moderate synergistic effect. The combination of Elderberry and Mulberry leaf had a CI value in the range of 0.9 to 1.1, suggesting a nearly additive effect.

### Example 5: Antiviral effects of combinations of extracts on SARS-CoV-2 virus infected Calu-3 cells

The same combinations as before (Mulberry leaf and Elderberry, Mulberry leaf and Quercetin (Quercefit), Elderberry and Quercetin (Quercefit)) have been examined for synergistic effects with SARS-CoV-2 virus (omicron BA.5 variant). The Calu-3 cells were infected with SARS-CoV-2 virus (omicron BA.5 variant) using the method as indicated in the Materials and Methods. Quercetin (Quercefit) 125 µg/ml, and extracts from Elderberry 833 µg/ml and Mulberry leaf 277 µg/ml stock solutions were prepared. The extracts were then subjected to 3-fold serial dilutions in the culture medium (8 dilution steps). Viral replication was determined by immunofluorescence of N protein as indicated in the Materials and Methods. The effects of the combined use were evaluated by calculating a Combination Index (CI) following the method of Chou, 2006.

Analysis of the data has been performed as described for H1N1. The effects of the combined use were evaluated by calculating a Combination Index (CI) following the method of Chou, 2006.

The results as presented in Figure 5 revealed the potential synergistic effect of Quercetin (Quercefit) with the extract from Elderberry with the tested plant extracts on inhibition of SARS-CoV-2 virus (omicron BA.5 variant) infectivity with the CI value of 0.42.

Figure 6 shows Calu-3 cells infected with SARS-CoV2 or mock-infected in the presence of the dilution steps shown in Figure 5 of elderberry extract, quercetin, or the combination of elderberry extract and quercetin as indicated. Cells were stained by immunofluorescence of the spike protein.

## Claims

1. A combination comprising a first component and a second component for use in the treatment or prevention of a respiratory tract infection, wherein said first component comprises quercetin, and said second component comprises at least one plant extract selected from the group consisting of Elderberry extract, Mulberry leaf extract, and combinations thereof.

2. The combination for use according to claim 1, which is a composition comprising said first component and said second component.

3. The combination for use according to claim 1, which is a kit comprising said first component and said second component.

4. The combination for use according to any one of claims 1 to 3, wherein said second component comprises Elderberry extract.

5. The combination for use according to claim 4, wherein said Elderberry extract is an extract from *Sambucus nigra* L. ssp. nigra, *Sambucus nigra* L. ssp. *canadensis,* and/or *Sambucus nigra* L. ssp. *cerulea.*

6. The combination for use according to any one of claims 1 to 5, wherein said second component comprises Mulberry leaf extract.

7. The combination for use according to claim 6, wherein said Mulberry leaf extract is an extract from *Morus alba, Morus nigra, Morus notabilis, Morus serrata, Morus celtidifolia, Morus insignis, Morus rubra,* and/or *Morus mesozygia.*

8. The combination for use according to any one of claims 1 to 7, wherein the respiratory tract infection is caused by an influenza A virus.

9. The combination for use according to claim 8, wherein the influenza A virus is H1N1, H2N2, or H3N2.

10. The combination of any one of claims 1 to 7, wherein the respiratory tract infection is caused by a coronavirus, optionally by SARS-CoV-2 virus.

11. The combination for use according to any one of claims 1 to 10, wherein said treatment or prevention comprises administering to a subject 100 mg to 1,000 mg of said quercetin.

12. The combination for use according to any one of claims 1 to 11, wherein said treatment or prevention comprises administering to a subject quercetin at a daily dose of 100 mg to 1,000 mg of said quercetin.

13. The combination for use according to any one of claims 1 to 12, wherein said treatment or prevention comprises administering to a subject 100 mg to 1,000 mg of mulberry leaf extract.

14. The combination for use according to any one of claims 1 to 13, wherein said treatment or prevention comprises administering to a subject a daily dose of 100 mg to 1,000 mg of mulberry leaf extract.

15. The combination for use according to any one of claims 1 to 14, wherein said treatment or prevention comprises administering to a subject 10 mg to 1,000 mg of elderberry extract.

16. The combination for use according to any one of claims 1 to 15, wherein said treatment or prevention comprises administering to a subject a daily dose of 10 mg to 1,000 mg of elderberry extract.

17. The combination for use according to any one of claims 1 to 16, wherein said treatment or prevention comprises administering said first component and said second component simultaneously.

18. The combination for use according to any one of claims 1 to 16, wherein said treatment or prevention comprises administering said first component and said second component sequentially.

19. The combination for use according to any one of claims 1 to 18, wherein said treatment or prevention comprises administering said first component and said second component for a period of 2 to 90 days.
